# EUROPEAN PATENT APPLICATION

(11) **EP 1 932 924 A1**
(43) Date of publication of application: **18.06.2008**
(21) Application number: 07022555.2
(22) Date of filing: 21.11.2007
(51) Int. Cl.: C12Q 1/68

(54) **Nucleic acid amplification method using microchip and microchip, and nucleic acid amplification system using the same**

(30) Priority: 22.11.2006 JP 2006316125; 16.11.2007 JP 2007298504
(71) Applicant: FUJIFILM Corporation, Minato-ku Tokyo (JP)
(72) Inventor: Iwaki, Yoshihide, Ashigarakami-gun Kanagawa (JP); Mori, Toshihiro, Ashigarakami-gun Kanagawa (JP)
(74) Representative: HOFFMANN EITLE

(57) **Abstract**

A nucleic acid amplification method, includes: performing nucleic acid amplification using a microchip that comprises: a specimen introduction section; a reaction section; and a channel that connects the reaction section and the specimen introduction section, wherein the method further comprises preventing a reaction solution from evaporating during the amplification reaction, and a microchip, includes: a specimen introduction section; a reaction section; and a channel that connects the reaction section and the specimen introduction section, wherein the microchip executes a method for preventing a specimen containing a nucleic acid from evaporating.

## Description

### Background of the Invention

### 1. Field of the Invention

This invention relates to a nucleic acid amplification method using a microchip and a microchip used for the nucleic acid amplification method, and a nucleic acid amplification system using the nucleic acid amplification method.

### 2. Description of the Related Art

In recent years, attention has been focused on a microchip (also called micro chemical device or microreactor) having a minute channel, namely, a microscale channel as an analysis method of a trace component in a trace sample.

The microchip of a minute chemical device having a channel of 1 mm or less in equivalent diameter sits in the limelight as a new trace analysis technology also satisfying rapidness and handiness from the points of being capable of lessening the sample amount and miniaturizing the device; particularly, application of the microchip to analysis of a trace component existing in blood, urine, a tissue, etc., is expected (for example, JP-A-2001-258868).

On the other hand, it is widely known that a nucleic acid is amplified to examine gene information of a trace sample. A nucleic acid is amplified in handling genes of DNA and RNA. To amplify a nucleic acid, an exponentially amplifying method like a PCR method is general and therefore the obtained nucleic acid sequence needs to exactly match the original nucleic acid sequence.

However, it is known that non-specific reaction also occurs in nucleic acid amplification reaction, and an attempt is made to make improvement (for example, JP-T-2003-525055 (the term "JP-T" as used herein means a published Japanese translation of a PCT patent application)).

### Summary of the Invention

To accomplish easy nucleic acid amplification from a trace sample, the inventors studied use of a microchip having a minute reaction system to perform nucleic acid amplification.

However, as a result of conducting nucleic acid amplification reaction in the microchip, surprisingly the inventors knew occurrence of non-specific amplification of a nucleic acid, and needed to find new countermeasures to prevent non-specific amplification.

It is therefore an object of the invention to provide a nucleic acid amplification method which is free of occurrence of non-specific amplification even if a microchip is used.

As a result of studying the causes of the above-mentioned problem, it is found that evaporation of a reaction solution during amplification reaction has something to do with non-specific amplification of a nucleic acid. It turned out that the obj ect is accomplished by using a method for preventing a reaction solution from evaporating during amplification reaction.

That is, the invention is embodied as the following configurations.
(1) A nucleic acid amplification method, comprising;
   performing nucleic acid amplification using a microchip that comprises: a specimen introduction section; a reaction section; and a channel that connects the reaction section and the specimen introduction section,
   wherein the method further comprises preventing a reaction solution from evaporating during the amplification reaction.
(2) The nucleic acid amplification method as described in (1) above,
   wherein a primer concentration during the amplification reaction is maintained 10 µM or less by preventing the reaction solution from evaporating during the amplification reaction.
(3) The nucleic acid amplification method as described in (1) or (2) above,
   wherein the nucleic acid amplification reaction is executed according to an isothermal amplification method.
(4) The nucleic acid amplification method as described in (3) above,
   wherein the nucleic acid amplification reaction according to the isothermal amplification method is executed at a temperature of 65°C or less.
(5) The nucleic acid amplification method as described in any of (1) to (4) above,
   wherein the reaction solution is prevented from evaporating by putting a lid on an opening of the specimen introduction section.
(6) The nucleic acid amplification method as described in (5) above,
   wherein the lid is a nonvolatile liquid.
(7) The nucleic acid amplification method as described in (6) above,
   wherein the nonvolatile liquid is a mineral oil.
(8) The nucleic acid amplification method as described in (5) above,
   wherein the lid is a lid using a material selected from the group consisting of a resin, a rubber and a glass, which are molded matching the opening of the specimen introduction section or a film.
(9) The nucleic acid amplification method as described in (8) above,
   wherein the lid is a lid using a thermoplastic resin or a photo-setting resin.
(10) The nucleic acid amplification method as described in any of (1) to (4) above,
   wherein the reaction solution is prevented from evaporating by keeping the specimen introduction section at a temperature of 35°C or less.
(11) A microchip, comprising:
   a specimen introduction section;
   a reaction section; and
   a channel that connects the reaction section and the specimen introduction section,
   wherein the microchip executes a method for preventing a specimen containing a nucleic acid from evaporating.
(12) The microchip as described in (11) above, which further comprises:
   a lid on an opening of the specimen introduction section.
(13) The microchip as described in (12) above,
   wherein the lid is a nonvolatile liquid.
(14) The microchip as described in (13) above,
   wherein the nonvolatile liquid is a mineral oil.
(15) The microchip as described in (12) above,
   wherein the lid is a lid using a material selected from the group consisting of a resin, a rubber and a glass, which are molded matching the opening of the specimen introduction section or a film.
(16) The microchip as described in (15) above,
   wherein the lid is a lid using a thermoplastic resin or a photo-setting resin.
(17) The microchip as described in (11) above, which further comprises:
   a heat regulation unit that keeps the specimen introduction section at a temperature of 35°C or less.
(18) A nucleic acid amplification system for performing nucleic acid amplification using a nucleic acid amplification method as described in any of (1) to (10) above.

### Brief Description of the Drawings

FIG. 1 is a schematic drawing to show an example of a channel in a microchip of the invention;
FIG. 2 is a graph to show the results of Example 1;
FIG. 3 is a graph to show the results of Example 1;
FIG. 4 is a graph to show the results of Example 1;
FIG. 5 is a graph to show the results of Example 1;
FIG. 6 is a schematic drawing (top view) to show a microchip used with Examples 2 to 4;
FIG. 7 is a graph to show the results of Example 2;
FIG. 8 is a schematic drawing to show heating conditions in Example 3;
FIG. 9 is a schematic drawing (perspective view) to show the microchip used with Examples 2 to 4; and
FIG. 10 is a drawing to show the positional relationships among primers,
wherein 1 denotes sample introduction section, 2 denotes channel, 3 denotes reaction section, 4 denotes 60°C heat regulation part, and 5 denotes 25°C heat regulation part.

### Detailed Description of the Invention

A nucleic acid amplification method of the invention is a method of performing nucleic acid amplification in a microchip having a specimen introduction section, a reaction section, and a channel for connecting the reaction section and the specimen introduction section, and is characterized by the fact that a method for preventing a reaction solution from evaporating during amplification reaction is used.

A microchip of the invention has a specimen introduction section, a reaction section, and a channel for connecting the reaction section and the specimen introduction section, and is characterized by the fact that it includes a method for preventing a reaction solution from evaporating during amplification reaction.

### <Nucleic acid amplification>

In the invention, although nucleic acid amplification is performed in a microchip, for preparations for a reaction system such as extraction of a nucleic acid and addition of a reaction substrate and reaction conditions, the nucleic acid amplification method can use the same reaction as in a conventional method.

As the nucleic acid amplification method, for example, PCR method (JP-B-4-67960, JP-B-4-67957), LCR method (JP-A-5-2934), SDA method (Strand Displacement Amplification: JP-A-5-130870), RCA method (Rolling Circle Amplification: Proc. Natl. Acad. Sci, vol. 92, 4641-4645 (1995)), ICAN method (Isothermal and Chimeric primer-initiated Amplification of Nucleic acids), LAMP method (Loop-Mediated Isothermal Amplification of DNA; Bio Industry, vol. 18, number 2 (2001)), NASBA method (Nucleic acid Sequence-based Amplification method; Nature, 350, 91- (1991)), TMA method (Transcription mediated amplification method; J. Clin Microbiol. vol. 31, 3270- (1993)), etc., can be named.

The most general and widespread nucleic acid amplification method is the PCR (polymerase chain reaction) method. The PCR method is a method of periodically controlling raising and lowering of the temperature of reaction liquid, thereby repeating the periodic steps of denaturation (step of denaturing a nucleic acid fragment from double strands to single strand) to annealing (step of hybridizing a primer in the nucleic acid fragment denatured to single strand) to polymerase (TaqDNA polymerase) elongation reaction to denaturing for amplifying the objective portion of the target nucleic acid fragment. Finally, the objective part of the target nucleic acid fragment can be amplified 1000,000 times the initial amount.

In the LCR method (JP-A-5-2934), two complementary oligonucleotide probe strands are joined to single stranded DNA in an end-to-tail manner and a nick between the two oligonucleotide strands is sealed with a heat resistant ligase. The joined DNA strand is liberated by heat denaturation and becomes a template and is amplified. As improvement in the LCR method, a method of setting a gap between two primers and filling in the gap with polymerase (Gap-LCR: Nucleic Acids Research, volume 23, number 4, 675- (1995)) is also developed.

The SDA method (JP-A-5-130870) is a cycling assay method using exonuclease and is one of amplification methods of the objective part of a target nucleic acid fragment using polymerase elongation reaction. This method is a method of decomposing a primer hybridized specifically in the objective part of a target nucleic acid fragment from an opposite direction by causing 5'→3' exonuclease to act together with polymerase elongation reaction with the primer as the starting point. A new primer in place of the decomposed primer is hybridized and again polymerase elongation reaction proceeds. The polymerase elongation reaction and decomposition reaction using exonuclease for removing the elongated strand are repeated in order periodically. The polymerase elongation reaction and the decomposition reaction using exonuclease can be executed under an isothermal condition.

The LAMP method is an amplification method of the objective part of a target nucleic acid fragment developed in recent years. This method is a method of amplifying the objective part of a target nucleic acid fragment under an isothermal condition as a special structure by using at least four types of primers for complementarily recognizing at least six specific parts of the target nucleic acid fragment and Bst DNA polymerase of strand displacement type having no nuclease activity in 5'→3' direction and catalyzing elongation reaction while liberating double stranded DNA on a template to single stranded DNA.

The ICAN method is also an amplification method of the objective part of a target nucleic acid fragment developed in recent years. It is an isothermal gene amplification method using an RNA-DNA chimeric primer, DNA polymerase having strand displacement activity and template exchange activity, and RNaseH. The chimeric primer is joined with a template and then a complementary strand is synthesized with the DNA polymerase. Then, RNaseH cuts the RNA portion derived from the chimeric primer and elongation reaction involving strand displacement reaction and template exchange reaction occurs from the cut portion; the reaction occurs repeatedly, whereby genes are amplified.

In every amplification method, non-specific amplification can occur as amplification starts with contamination caused by bringing an amplification product into a reaction solution or forming of a dimer by the same or different primers as a trigger.

Then, to prevent primers from forming a dimmer, a primer sequence needs to be considered for designing or it is necessary to prevent the primer concentration in reaction liquid from being set to a too high concentration. Preferably, the primer concentration is 0.2 to 1.0 µM in total of two primers for PCR; preferably the primer concentration is 4 to 9 µM in total of all primers for isothermal amplification of the LAMP method, etc.

With the isothermal amplification method, often a primer is added at a higher concentration than in usual PCR to enhance the reaction efficiency. Thus, as evaporation of liquid occurs during amplification reaction, the primer is concentrated and a dimmer is easily formed. Then, it is important to devise a method of preventing evaporation of liquid occurs during amplification reaction so that the primer is not concentrated.

As other reagents used for nucleic acid amplification, reagents used for known nucleic acid amplification methods such as isothermal amplification reaction of LAMP amplification reaction, etc., the polymerase amplification reaction of general PCR amplification reaction, etc., and ligase amplification reaction of LCR, etc., can be used.

Specifically, polymerase and/or ligase (preferably, a heat resistant enzyme of Taq polymerase, etc.,), a nucleic acid substrate (dATP/dTTP (dUTP)/dCTP/dGTP dNTP), buffer liquid (for example, Tris-HC1, etc.,), a melting temperature regulator (for example, DMSO), etc., suited to them, and the like can be used; they can be used with similar type, amount, and method to those of conventional nucleic acid amplification.

In the invention, use of the isothermal nucleic acid amplification method of the LAMP method, etc., capable of executing reaction at a given temperature is preferred to use of the PCR method in the related art requiring temperature change in response to the amplification reaction cycle from the viewpoints of ease of handling and use of a small chip.

The term "isothermal" mentioned here refers to keeping under an almost constant temperature condition that an enzyme and a primer can function substantially. The expression "almost constant temperature condition" is used not only to mean keeping the setup temperature precisely, but also to mean that temperature change to such an extent that the substantial function of an enzyme and a primer is not impaired is allowed; for example, temperature change of about 10°C would be allowed.

In the invention, "nucleic acid" is not limited and DNA and RNA or any other artificial nucleic acid (PNA, etc.,) can be used; for example, it may be a natural specimen of cDNA, genomic DNA, mRNA, etc., or may be synthetic polynucleotide. A double stranded nucleic acid, a straight-chain nucleic acid, and a cyclic nucleic acid are contained.

### <Microchip>

In the invention, the microchip is a microchip having at least a specimen introduction section, a reaction section, and a channel for connecting the reaction section and the specimen introduction section, and the like. Specifically, it has a channel of 1 mm or less in equivalent diameter.

The equivalent diameter mentioned in the invention is also called equilibrium and is a term used generally in the mechanical engineering field. Assuming an equivalent circular pipe for piping which is any shape in cross section (corresponding to channel in the invention), the diameter of the equivalent circular pipe is referred to as equivalent diameter and equivalent diameter deq is defined as deq = 4A/p where A is the cross-sectional area of piping and p is the wetted perimeter length (peripheral length) of piping. To apply the equivalent diameter to a circular pipe, the equivalent diameter matches the diameter of the circular pipe. The equivalent diameter is used to estimate the flow or heat transmission characteristic of the piping based on data of the equivalent circular pipe and represents the spatial scale (representative length) of a phenomenon. The equivalent diameter becomes deq = 4a²/4a = a in a square tube with each side a or becomes deq = 2h in a flow between parallel flat plates having path height h. The detailed description of this topic is given in "Kikai kougaku jiten" ((Sha) Nihon Kikai Gakkai hen 1997, Maruzen (Kabu)).

The equivalent diameter of the channel used in the invention is 1 mm or less; preferably it is 10 to 500 µm and particularly preferably it is 20 to 300 µm.

The length of the channel is not limited; preferably it is 1 mm to 10000 mm and particularly preferably it is 5 mm to 100 mm.

The width of the channel used in the invention preferably is 1 to 3000 µm; more preferably 10 to 2000 µm; furthermore preferably 50 to 1000 µm. If the width of the channel is in the range, degradation of flowability as a sample receives resistance from the wall of the channel is small and the sample amount can be kept small.

The channel may be only one channel or may be two or more branches conforming to the number of elements placed in the microchip. It can be any form such as a line or a curve; preferably it is shaped like a line.

The channel of the microchip can be prepared on a solid substrate according to micromachining technology.

As an example of a material used as the solid substrate, metal, silicon, Teflon (registered trademark), glass, ceramics, plastics, etc., can be named. Among them, metal, silicon, Teflon, glass, and ceramics are preferred from the viewpoints of heat resistance, pressure resistance, solvent resistance, and light transparency; glass is particularly preferred.

As the micromachining technology to prepare a channel, for example, methods described in "Microreactor - Shinjidaino gouseigijyutu - (2003, published by CMC, supervised by Yoshida Jyunichi Kougaku kenkyuuka professor at the graduate school at the Kyouto University), "Bisaikakou gijyutu ouyouhen - Photonics electronics mechatronics heno ouyou - (2003, published by NTS, edited by Koubunshi gakkai gyouji iinkai), etc.

Representative methods are as follows: LIGA technology using X-ray lithography, a high aspect ratio photolithography method using EPON SU-8, a micro electrical discharge machining method (µ-EDM), a high aspect ratio machining method of silicon based on Deep RIE, a Hot Emboss machining method, a stereo lithography method, a laser machining method, an ion beam machining method, a mechanical micro cutting method using a microtool made of a hard material such as diamond, and the like. The technologies may be used singly or in combination. The preferred micromachining technology are the LIGA technology using X-ray lithography, the high aspect ratio photolithography method using EPON SU-8, the micro electrical discharge machining method (µ-EDM), and the mechanical micro cutting method.

The channel used in the invention can also be prepared by using a pattern formed using a photo resist on a silicon wafer as a mold and pouring a resin into the mold and solidifying (molding method). The molding method can use a silicon resin represented by polydimethylsiloxane (PDMS) or a derivative thereof.

To assemble the micro chemical device of the invention, welding technique can be used. The usual welding technique is roughly classified into solid-phase welding and liquid-phase welding; as generally used welding methods, pressure welding and diffusion welding as the solid-phase welding and welding, eutectic bonding, soldering, adhesion, etc., as the liquid-phase welding are representative welding methods.

Further, at the assembling time, it is desirable that a highly accurate welding method keeping the dimension accuracy not involving destruction of the minute structures of the channel, etc., caused by deterioration or large deformation of material by heating at a high temperature should be adopted; as the technique, silicon direct welding, anode welding, surface activation welding, direct welding using hydrogen bonding, welding using an HF water solution, Au-Si eutectic bonding, bond free adhesion, etc., can be named.

A specimen containing a nucleic acid moves from the specimen introduction section through the channel to the reaction section. As a method of moving the specimen through the channel, preferably a continuous flow method, a droplet (liquid plug) method, a drive method, etc., or use of a capillary phenomenon is used.

In fluid control of the continuous flow method, the inside of the channel of the microchip needs to be full of fluid and it is a common practice to drive the whole fluid by a pressure source of an externally provided syringe pump, etc. The continuous flow method makes it possible to implement a control system according to comparatively easy setup.

The droplet (liquid plug) method is to move a droplet separated by air in the chip inside or the channel to the chip, and each droplet is driven by an air pressure. In the droplet method, a vent structure for letting air between a droplet and a channel wall or droplets escape to the outside as required, a valve structure to keep pressure in a branch channel independently of other portions, and the like need to be provided in the inside of the chip. To control a pressure difference for manipulating a droplet, it is necessary to construct an external pressure control system made up of a pressure source and a switch valve. The droplet method is preferred because multi-step manipulation is possible in such a manner that a plurality of droplets are handled separately and several reactions are executed in order and the flexibility of the system configuration is increased.

Generally widely used as the drive method are an electric drive method of applying a high voltage across a channel to generate an electroosmotic flow, thereby moving fluid, a pressure drive method of providing an external pressure source and applying a pressure to a fluid for moving the fluid, and a drive method using a capillary phenomenon.

In the electric drive method, it is known that a flow speed profile becomes a flat distribution as fluid behavior in the channel cross section. In the pressure drive method, it is known that a flow speed profile becomes like a hyperbola, namely, becomes a distribution wherein the flow speed is high in the channel center and is low in a wall surface portion as fluid behavior in the channel cross section. Thus, the electric drive method is preferred for the purpose of moving fluid with the shape of a sample plug, etc., kept.

In the electric drive method, the inside of the channel needs to be full of fluid, namely, the electric drive method takes the mode of the continuous flow method. Since fluid manipulation can be performed under electric control, comparatively complicated processing can be performed, for example, in such a manner that a concentration slope with time is made by continuously changing the mix ratio of two types of solutions.

In the pressure drive method, control is possible without being affected by the fluid-proper electric nature. The application range of the pressure drive method is wide because an accessory effect of heat generation, electrolysis, etc., need not be considered and almost no effect on the substrate exists. The pressure drive method requires that an eternal pressure source be provided.

The invention enables the user to select the sample moving method as required conforming to the type of sample, the inspection item, etc. Among the methods, the droplet (liquid plug) method or the drive method using a capillary phenomenon is preferred. More preferably, the air pressure in the droplet (liquid plug) method is placed in a negative pressure; particularly preferably the air pressure is placed in a negative pressure by suction of air.

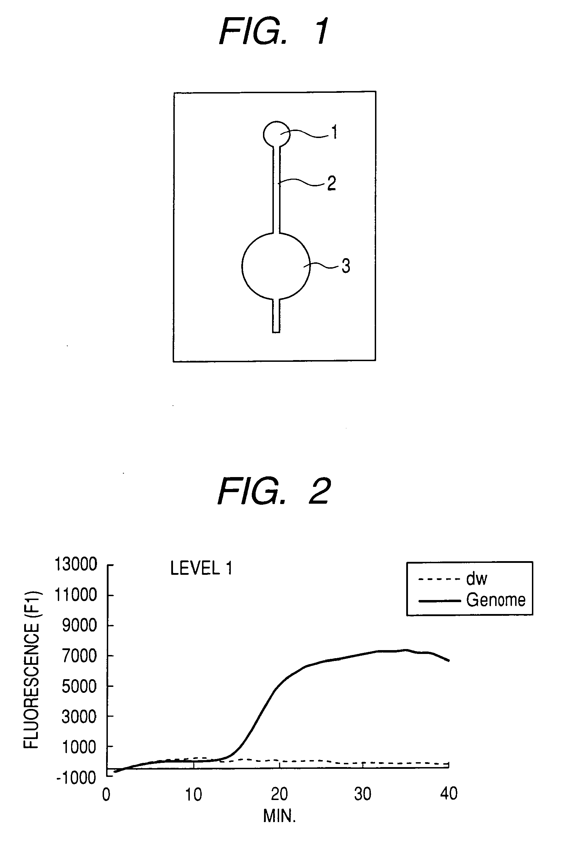

FIG. 1 shows the simplest embodiment.

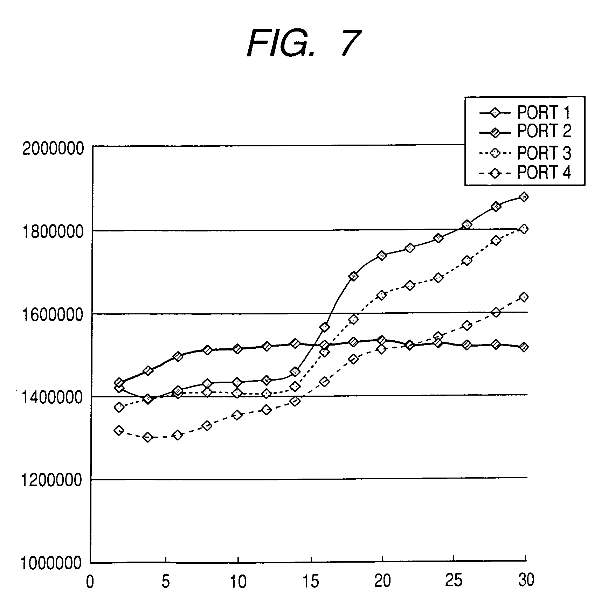

A specimen introduction section 1 may be any if it enables a sample to be poured. The poured sample passes through a channel 2 and is guided into a reaction section 3. In the reaction section 3, nucleic acid amplification reaction is executed. One chip may contain a plurality of cells (reaction paths each containing the specimen introduction section 1, the channel 2, and the reaction section 3) as shown in FIG. 6 and one cell may have two or more specimen introduction sections 1; preferably one of them is used as an air vent.

### <Method for preventing a reaction solution from evaporating during amplification reaction>

The nucleic acid amplification method of the invention is characterized by the fact that a method for preventing a reaction solution from evaporating during amplification reaction is used when nucleic acid amplification is performed in the above-described microchip.

More preferably, evaporation of a reaction solution during amplification reaction is prevented.

As a method for preventing a specimen containing a nucleic acid from evaporating, for example, a lid can be put on an opening of the specimen introduction section, etc., of the microchip. Specifically, sealing with nonvolatile liquid such as mineral oil, a lid using a material of resin, rubber, glass, etc., molded matching the opening of the specimen introduction section, etc., hermetic sealing by putting a film, etc., on the opening, or the like can be named. For example, sealing with thermoplastic resin or photo-setting resin is also preferred.

To prevent evaporation, execution of nucleic acid amplification reaction at a low temperature is also effective method. Specifically, although heating to 90°C or more is repeated in general PCR reaction, if the LAMP method, etc., is used, reaction can be executed with the temperature kept at 70°C or less and the LAMP method, etc., is preferred for preventing evaporation.

Further, to prevent a reaction solution from evaporating during amplification reaction, it is also preferable to maintain only the reaction section at a temperature suited to nucleic acid amplification reaction (about 40°C to 98°C; more preferably 45°C to 75°C; furthermore preferably 55°C to 65°C) and keep other portions (particularly the specimen introduction section) at a low temperature (for example, 35°C or less; more preferably 30°C or less).

Evaporation of a reaction solution during amplification reaction can also be prevented by designing the shape of the chip. For example, in FIG. 1, the diameter of the channel 2 leading to the reaction section 3 is narrowed or the channel is meandered, whereby evaporation from the channel 2 to the specimen introduction section 1 can be prevented.

### <Nucleic acid amplification system>

In the invention, the nucleic acid amplification system is intended for a system for fully automatically performing operation from sample preparation to amplification, used in a clinical assay to detect an abnormal gene in an individual and to detect pathogenic microorganisms in a sample, etc. A general amplification system in the invention includes the microchannel chip, an inspection apparatus for supplying liquid to the chip and executing inspection, and a nucleic acid amplification reagent kit.

The inspection apparatus is provided with a stage for placing the microchannel chip, a move unit that moves liquid through a channel in the microchannel chip, a heating unit that heats the liquid in the microchannel chip to cause a chemical reaction to occur at an appropriate temperature, a detection unit that detects the reaction using a detection reagent (for example, a fluorescent pigment, etc.,) contained in an inspection reagent, and a determination unit that determines the result according to information of the sample detected by the detection unit.

The nucleic acid amplification reagent kit contains substances required for amplifying a specimen nucleic acid. The substances include a reaction substrate of dNTP, its derivative, etc., bivalent ions of Mg²⁺, etc., a primer for defining the gene corresponding to an inspection item, a nucleic synthesis enzyme required for the amplification reaction, a buffer for determining a reaction optimum condition of the nucleic synthesis enzyme, etc. The substances may include a melting point adjustor of DMSO, etc., and an enzyme stabilizer of BSA, etc., in some cases. The reagent kits can be previously carried in the channel in the microchannel chip or can be provided aside from the microchannel chip.

The simplest embodiment is shown. To use the microchip in FIG. 1 as the microchannel chip, first a sample is poured into the specimen introduction section 1. At this time, an amplification reagent is previously carried in the reaction section of the microchannel chip or is poured into the specimen introduction section 1 together with the sample. Next, using the move unit of the inspection apparatus, the poured specimen is moved through the channel 2 to the reaction section 3. The move unit can be appropriately set conforming to the sample type, the inspection item, etc., as described above. Then, the neighbor of the reaction section 3 is heated to an appropriate temperature using the heating unit of the inspection apparatus. For the heating method, a heat block, heat convection, etc., can also be selected appropriately. Further, the detection reagent previously contained in the reagent is detected in a given time using the detection unit of the inspection apparatus. For example, for nucleic acid amplification, detecting of a double stranded nucleic acid using a fluorescent intercalater of SYBR Green I, etc., is widely known. In this case, the detection reagent and the detection unit can also be selected appropriately. The nucleic acid amplification method may be any if it is an isothermal amplification method.

Since the protocol is automated, the advantages of the amplification system is as follows: (1) Suitability for setting of a clinical laboratory, (2) capability of giving the controlled and consistent (standardizable) result, (3) capability of quantifying a nucleic acid in a specific sample, (4) capability of detecting and quantifying a large number of target nucleic acids in a test sample, (5) high-sensitive and efficient detection capability of a nucleic acid in a serum sample and on the spot, and (6) enhancing of capability of detecting a single mutation in a target. Further, the amplification system is characterized by the fact that it includes a measure for preventing the reaction solution from evaporating during amplification reaction described later. According to the features, a routine diagnostic test can be conducted easily, rapidly, and precisely.

### [Example 1]

The invention will be discussed below more specifically with examples, but the scope of the invention is not limited to the examples.

### <Example 1> Evaluation of non-specific amplification based on presence or absence of evaporation using LAMP method

### (1) Preparation of nucleic acid specimen liquid containing a target nucleic acid fragment

Human Genomic DNA (manufactured by Clontech) 100 ng was heated for 3 min at 98°C and was denatured to a single strand and then a sequence in β-actin gene was amplified under the following conditions to obtain nucleic acid specimen liquid (Genome). As negative control, a sample was also prepared by heating sterile water (dw) under a condition similar to that described above.

### <Primer>

Primers were designed with the β actin gene as a target so that basically the LAMP method can be used. A forward primer was designed so that a 3'termination region complementary to a template and a 5' termination region are hybridized with a region 10 bases downstream from the 3' termination base on the elongation strand of the primer, and four bases of T were intervened in concatenating the 5' termination region and the 3' termination region. A reverse primer was designed so that a 3'termination region complementary to a template and a 5' termination region are hybridized with a region 22 bases downstream from the 3' termination base on the elongation strand of the primer, and four bases of T were intervened in concatenating the 5' termination region and the 3' termination region.

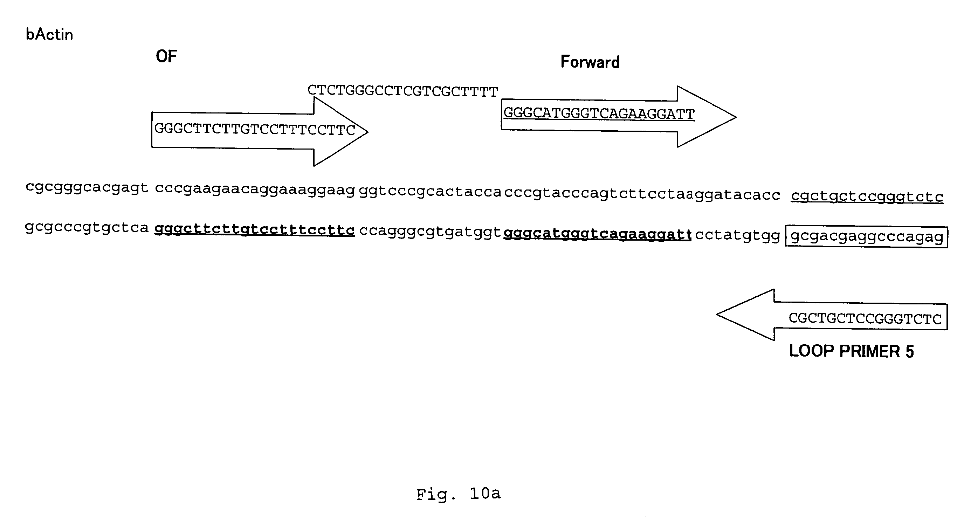

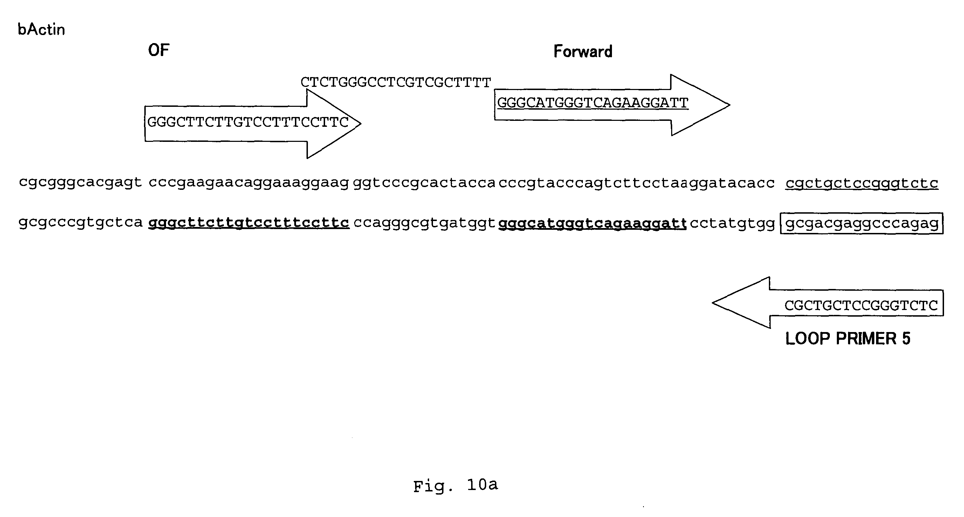

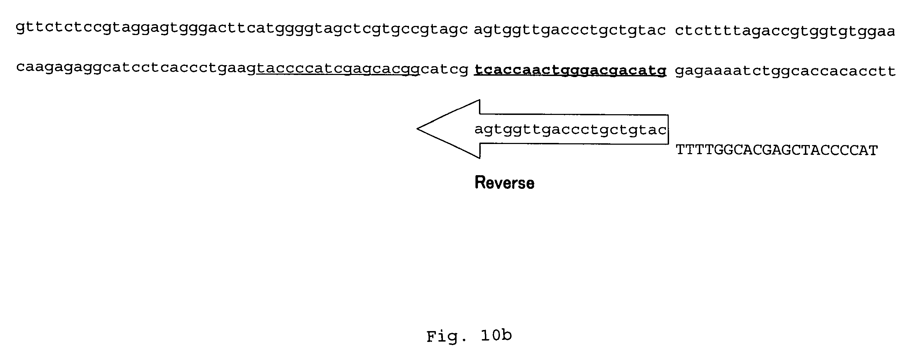

Outer primers (OF and OR) were designed on the outsides of the forward primer and the reverse primer. The following primer was used as a loop primer. FIG. 10 is a drawing to show the positional relationships among the primers.
Primer 1 (Forward):
   5'-CTCTGGGCCTCGTCGCTTTTGGGCATGGGTCAGAAGGATT-3' (SEQ ID NO:1)
Primer 2 (Reverse):
   5'-TACCCCATCGAGCACGGTTTTCATGTCGTCCCAGTTGGTGA-3' (SEQ ID NO:2)
Outer primer 3 (OF)
   5'-GGGCTTCTTGTCCTTTCCTTC-3' (SEQ ID NO:3)
Outer primer 4 (OR)
   5'-CCACACGCAGCTCATTGTAG-3' (SEQ ID NO:4)
Loop primer 5
   5' -CGCTGCTCCGGGTCTC-3' (SEQ ID NO:5)

### (2) Forcible evaporation experiment

Liquid of the following composition was added to a tube and was heated for given times (0 min, 5 min, 10 min, and 20 min) at 98°C with a lid of the tube open to forcibly evaporate the reaction liquid. Then, the remaining amount at each level was checked and then a predetermined amount of enzymes (Bst. Polymerase and Taq-MutS) was added to a solution at each level.

### <Composition of reaction liquid>

| | |
|---|---|
| 10 x Bst Buffer (DF) | 2.5 µL |
| 100 mM MgSO₄ | 1.5 µL |
| 10% (v/v) Tween 20 | 0.25 µL |
| 100% DMSO | 1.25 µL |
| 25 mM dNTP each | 1.4 µL |
| SYBR Green I | 0.5 µL |
| Primer 1 (50 µM) | 1.6 µL |
| Primer 2 (50 µM) | 1.6 µL |
| Primer 3 (50 µM) | 0.2 µL |
| Primer 4 (50 µM) | 0.2 µL |

### Nucleic acid specimen liquid or sterile water

| | 1.0 µL (nucleic acid 100 ng) |
|---|---|
| Purified water | 11.0 µL |
| Total | 23.0 µL |

The obtained reaction liquid was heated under the conditions of no heating (experiment level 1), heating for five minutes at 98°C (experiment level 2), heating for 10 minutes at 98°C (experiment level 3), and heating for 20 minutes at 98°C (experiment level 4). Then, the remaining amount of the specimen under each condition was checked.

Next, enzymes (Bst. Polymerase 1.0 µL and TaqMutS 1.0 µL) were added to the specimen under each condition.

### (3) Nucleic acid amplification reaction

Amplification reaction was executed by causing the solution under each condition mentioned above to react for 30 minutes at 60°C.

### (4) Detection of amplification reaction

For the amplification reaction in (3), fluorescence detection was conducted using a real-time fluorescence detector (Mx3000p, manufactured by Stratagene).

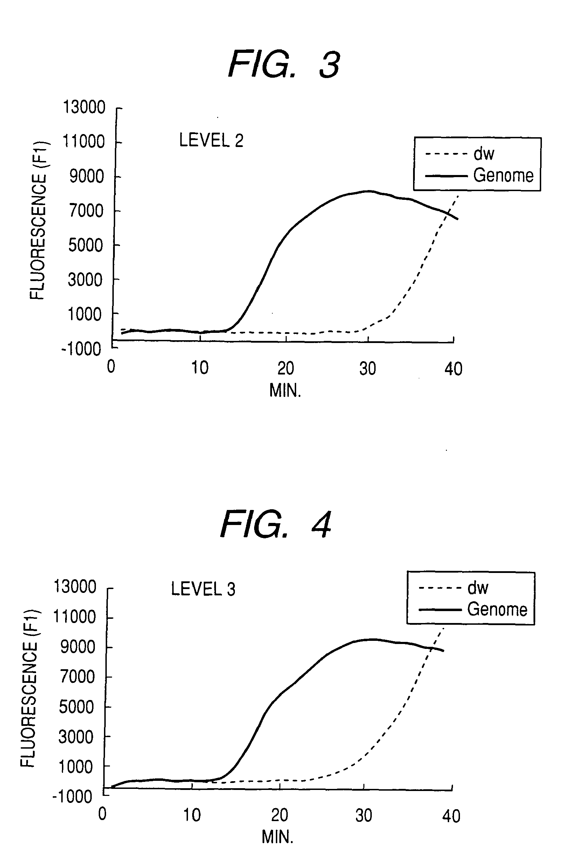

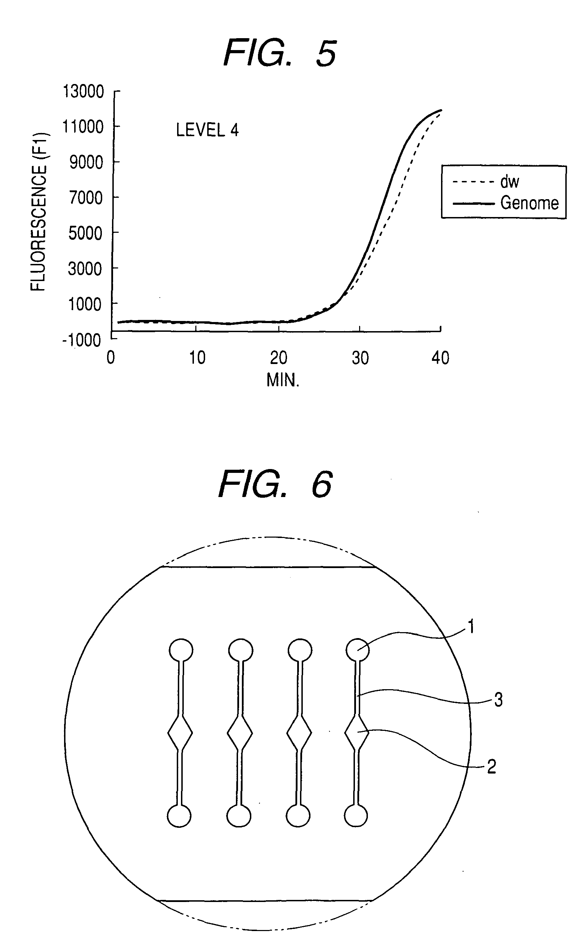

The results are shown as graphs in FIGS. 2 to 5.

From the results, it is seen that non-specific amplification from water occurs by prolonging the heating time at 98°C from level 1 to level 4, namely, as the evaporation amount is larger. As for level 4, amplification from genome and amplification from water cannot be distinguished from each other. The time when the fluorescence amount reaches 250 in the graphs was calculated using analysis software of Mx3000p. At the same time, the evaporation amount and the substantial primer concentration calculated from the evaporation amount at each level are also shown. There was no difference in the evaporation amount in the cases of sterile water specimen and nucleic acid specimen.

Table 1 lists the result. In the table, No Ct means no occurrence of amplification.

**Table 1**

| | Evaporation amount | Substantial primer concentration | Nucleic acid specimen (min) | Sterile water specimen (min) |
|---|---|---|---|---|
| Level 1 | 0 µL | 7.2 µM | 14.1 | No Ct |
| Level 2 | 4 µL | 8.6 µM | 13.5 | 29.5 |
| Level 3 | 9 µL | 11.25 µM | 13.5 | 24.7 |
| Level 4 | 13 µL | 15.0 µM | 22.7 | 23.7 |

From Table 1, it is seen that amplification reaction from water is easy to occur in response to the evaporation amount. This corresponds to a point where 8.6 µM is exceeded in terms of the substantial primer concentration.

### <Example 2> Evaluation of non-specific amplification based on presence or absence of evaporation on chip using LAMP method

### (hermetic sealing with mineral oil)

### (1) Preparation of nucleic acid specimen liquid containing a target nucleic acid fragment, primer

Nucleic acid specimen liquid and sterile water (dw), primer were prepared in a similar manner to that in Example 1.

### (2) Nucleic acid amplification reaction

Amplification reaction was executed by introducing the following reaction liquid into the following chip and causing the reaction liquid to react for 30 minutes at 60°C.

### <Composition of reaction liquid>

| | |
|---|---|
| 10 x Bst Buffer (DF) | 2.5 µL |
| 100 mM MgSO₄ | 1.5 µL |
| 10% (v/v) Tween 20 | 0.25 µL |
| 100% DMSO | 1.25 µL |
| 25 mM dNTP each | 1.4 µL |
| SYBR Green I (50 µM) | 0.5 µL |
| Primer 1 (50 µM) | 1.6 µL |
| Primer 2 (50 µM) | 1.6 µL |
| Primer 3 (50 µM) | 0.2 µL |
| Primer 4 (50 µM) | 0.2 µL |
| Bst. Polymerase | 1.0 µL |
| TaqMutS | 1.0 µL |

| Nucleic acid specimen liquid or sterile water | |
|---|---|
| | 1.0 µL (nucleic acid 100 ng) |
| Purified water | 11.0 µL |
| Total | 25.0 µL |

### (3) Fabrication of evaluation chip

Evaporation was evaluated using the chip shown in FIG. 6. Four cells were under conditions listed below in Table 2. After reaction liquid was introduced from one of specimen introduction sections (1), the two cells were sealed as each cell fills at both ends (two specimen introduction sections (1)) with mineral oil (manufactured by Sigma) after the reaction liquid was added to take a measure against evaporation. A measure against evaporation is not taken for the remaining two cells and amplification reaction was conducted in the cells in a state in which the above-mentioned reaction liquid was added.

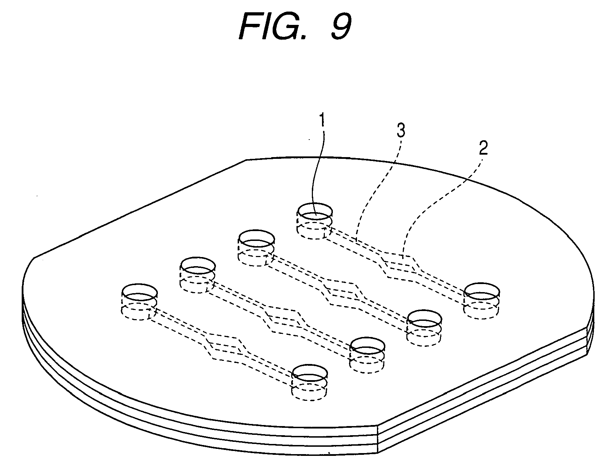

FIG. 6 is a top view; numeral 1 denotes the specimen introduction section, numeral 2 denotes the reaction section, and numeral 3 denotes the channel. FIG. 9 is a perspective view of the chip in FIG. 6 and the reference numerals denote the same parts as in FIG. 6.

### (4) Detection of amplification reaction

To detect amplification reaction, a photograph was taken every two minutes using LAS-3000 (manufactured by FUJIFIM Corporation). Next, the fluorescence value of each cell was quantified using analysis software MultiGauge (manufactured by FUJIFIM Corporation). The results are shown in Table 2 and FIG. 7.

**Table 2**

| | Measure against evaporation | Specimen | Amplification |
|---|---|---|---|
| Cell 1 | Included | Nucleic acid specimen | Executed |
| Cell 2 | | Sterile water | None |
| Cell 3 | None | Nucleic acid specimen | Executed |
| Cell 4 | | Sterile water | Executed |

From the results, it is seen that non-specific amplification is suppressed by setting a situation in which evaporation does not occur.

### <Example 3> Evaluation of non-specific amplification based on presence or absence of evaporation on chip using LAMP method

### (temperature adjustment)

Nucleic acid specimen liquid and sterile water (dw), primer were prepared and was introduced into the following chip and nucleic acid amplification was executed in a similar manner to that in Example 2.

### Fabrication of evaluation chip

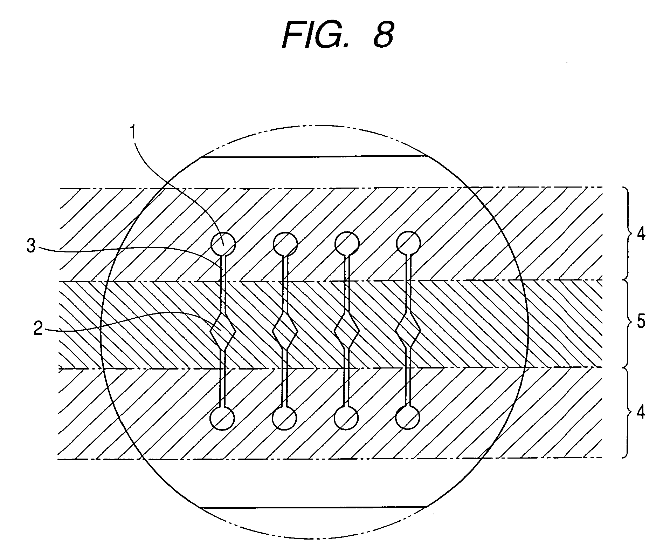

Evaporation was evaluated using the chip shown in FIG. 6. Two chips were used. In one chip (cells 5 to 8), amplification reaction was executed for 30 minutes on a heat regulation unit capable of heating the full face of the chip at 60°C. In the other chip (cells 9 to 12), amplification reaction was executed for 30 minutes on a unit having a heat regulation unit for heating a center (5) of the chip at 60°C and a heat regulation unit capable of maintaining the temperature of peripheries (4) at 25°C as a measure against evaporation, as shown in FIG. 8. The four cells in each chip were under the conditions in Table 3 given below.

### (4) Detection of amplification reaction

To detect amplification reaction, a photograph was taken every two minutes using LAS-3000 (manufactured by FUJIFILM Corporation). Next, the fluorescence value of each cell was quantified using analysis software MultiGauge (manufactured by FUJIFILM Corporation). The results are listed below:

**Table 3**

| | Measure | Sample | Amplification |
|---|---|---|---|
| Cell 5 | None (full face) | Nucleic acid specimen | Executed |
| Cell 6 | None (full face) | Nucleic acid specimen | Executed |
| Cell 7 | None (full face) | Sterile water | Executed |
| Cell 8 | None (full face) | Sterile water | Executed |
| Cell 9 | Included (FIG. 8) | Nucleic acid specimen | Executed |
| Cell 10 | Included (FIG. 8) | Nucleic acid specimen | Executed |
| Cell 11 | Included (FIG. 8) | Sterile water | None |
| Cell 12 | Included (FIG. 8) | Sterile water | None |

From the results, it is seen that non-specific amplification is suppressed by setting a situation in which evaporation does not occur.

### <Example 4> Evaluation of non-specific amplification based on presence or absence of evaporation on chip using LAMP method

### (hermetic sealing with UV-setting resin)

Nucleic acid specimen liquid and sterile water (dw), primer were prepared and was introduced into the following chip and nucleic acid amplification was executed in a similar manner to that in Example 2.

### Fabrication of evaluation chip

Amplification reaction was executed using the chip shown in FIG. 6 (the full face of the chip was heated at 60°C). The four cells were placed at levels in Table 4 given below. In each of two cells, a drop of UV-setting resin (8815K, Kyouritu Kagaku Sangyou) was added to both sides of sample instruction section (1) and UV irradiation was executed for 30 seconds from a height of 1 cm above the cell from a UV light source (400 nm) for sealing the port to take a measure against evaporation. For the remaining two cells, no measure against evaporation is taken and amplification reaction was executed for 30 minutes in a state in which the above-mentioned reaction liquid is only added.

### (4) Detection of amplification reaction

To detect amplification reaction, a photograph was taken every two minutes using LAS-3000 (manufactured by FUJIFILM Corporation). Next, the fluorescence value of each cell was quantified using analysis software MultiGauge (manufactured by FUJIFILM Corporation). The results are listed below:

**Table 4**

| | Measure | Specimen | Amplification |
|---|---|---|---|
| Cell (port) 1 | Included | Nucleic acid specimen | Executed |
| Cell (port) 2 | | Sterile water | None |
| Cell (port) 3 | None | Nucleic acid specimen | Executed |
| Cell (port) 4 | | Sterile water | Executed |

From the results, it is seen that non-specific amplification is suppressed by setting a situation in which evaporation does not occur.

According to the invention, there is provided the nucleic acid amplification method which is free of occurrence of non-specific amplification even if a microchip is used. Accordingly, it is made possible to perform precise nucleic acid amplification in a short time according to an easy method from a trace sample.

The entire disclosure of each and every foreign patent application from which the benefit of foreign priority has been claimed in the present application is incorporated herein by reference, as if fully set forth.

## Claims

1. A nucleic acid amplification method, comprising:
performing nucleic acid amplification using a microchip that comprises: a specimen introduction section; a reaction section; and a channel that connects the reaction section and the specimen introduction section,
wherein the method further comprises preventing a reaction solution from evaporating during the amplification reaction.

2. The nucleic acid amplification method according to claim 1,
wherein a primer concentration during the amplification reaction is maintained 10 µM or less by preventing the reaction solution from evaporating during the amplification reaction.

3. The nucleic acid amplification method according to claim 1,
wherein the nucleic acid amplification reaction is executed according to an isothermal amplification method.

4. The nucleic acid amplification method according to claim 3,
wherein the nucleic acid amplification reaction according to the isothermal amplification method is executed at a temperature of 65°C or less.

5. The nucleic acid amplification method according to claim 1,
wherein the reaction solution is prevented from evaporating by putting a lid on an opening of the specimen introduction section.

6. The nucleic acid amplification method according to claim 5,
wherein the lid is a nonvolatile liquid.

7. The nucleic acid amplification method according to claim 6,
wherein the nonvolatile liquid is a mineral oil.

8. The nucleic acid amplification method according to claim 5,
wherein the lid is a lid using a material selected from the group consisting of a resin, a rubber and a glass, which are molded matching the opening of the specimen introduction section or a film.

9. The nucleic acid amplification method according to claim 8,
wherein the lid is a lid using a thermoplastic resin or a photo-setting resin.

10. The nucleic acid amplification method according to claim 1,
wherein the reaction solution is prevented from evaporating by keeping the specimen introduction section at a temperature of 35°C or less.

11. A microchip, comprising:
a specimen introduction section;
a reaction section; and
a channel that connects the reaction section and the specimen introduction section,
wherein the microchip executes a method for preventing a specimen containing a nucleic acid from evaporating.

12. The microchip according to claim 11, which further comprises;
a lid on an opening of the specimen introduction section.

13. The microchip according to claim 12,
wherein the lid is a nonvolatile liquid.

14. The microchip according to claim 13,
wherein the nonvolatile liquid is a mineral oil.

15. The microchip according to claim 12,
wherein the lid is a lid using a material selected from the group consisting of a resin, a rubber and a glass, which are molded matching the opening of the specimen introduction section or a film.

16. The microchip according to claim 15,
wherein the lid is a lid using a thermoplastic resin or a photo-setting resin.

17. The microchip according to claim 11, which further comprises:
a heat regulation unit that keeps the specimen introduction section at a temperature of 35°C or less.

18. A nucleic acid amplification system for performing nucleic acid amplification using a nucleic acid amplification method according to claim 1.


SEQUENCE LISTING

<110>  Fuji Film Co. Ltd.,

<120>  The primer for gene amplification

<130>  206-02377

<160>  6

<170>  PatentIn version 3.1

<210>  1
<211>  40
<212>  DNA
<213>  Artificial

<220>
<223>  Artificially synthesized oligonucleotide sequence

<400>  1
ctctgggcct cgtcgctttt gggcatgggt cagaaggatt                                40

<210>  2
<211>  29
<212>  DNA
<213>  Artificial

<220>
<223>  Artificially synthesized oligonucleotide sequence

<400>  2
acatgttttc atgtcgtccc agttggtga                                            29

<210>  3
<211>  21
<212>  DNA
<213>  Artificial

<220>
<223>  Artificially synthesized oligonucleotide sequence

<400>  3
gggcttcttg tcctttcctt c                                                    21

<210> 4
<211> 20
<212> DNA
<213> Artificial

<220>
<223> Artificially synthesized oligonucleotide sequence

<400> 4
ccacacgcag ctcattgtag 20


<210> 5
<211> 16
<212> DNA
<213> Artificial

<220>
<223> Artificially synthesized oligonucleotide sequence

<400> 5
ctctgggcct cgtcgc 16


<210> 6
<211> 213
<212> DNA
<213> Homo sapiens
<220>
<223> Beta-Actin sequence

<400> 6
cgcgggcacg agtcccgaag aacaggaaag gaagggtccc gcactaccac ccgtacccag 60
tcttcctaag gatacacccg ctgctccggg tctcgttctc tccgtaggag tgggacttca 120
tggggtagct cgtgccgtag cagtggttga ccctgctgta cctcttttag accgtggtgt 180
ggaagatgtt actcgacgca caccgagggc tcc 213


<210> 7
<211> 213
<212> DNA
<213> Homo sapiens
<220>
<223> Beta-Actin sequence

<400> 7

gcgcccgtgc tcagggcttc ttgtcctttc cttcccaggg cgtgatggtg ggcatgggtc 60
agaaggattc ctatgtgggc gacgaggccc agagcaagag aggcatcctc accctgaagt 120
accccatcga gcacggcatc gtcaccaact gggacgacat ggagaaaatc tggcaccaca 180
ccttctacaa tgagctgcgt gtggctcccg agg 213